(19) Europäisches Patentamt
European Patent Office
Office européen des brevets

(11) Numéro de publication: 0 371 876 B1

(12) **FASCICULE DE BREVET EUROPEEN**

(45) Date de publication de fascicule du brevet: **24.11.93**

(51) Int. Cl.5: **C07D 261/18**, A61K 31/42, C07D 261/14, C07D 261/08, C07D 261/20, C07D 261/04

(21) Numéro de dépôt: **89403300.0**

(22) Date de dépôt: **28.11.89**

(54) **Composés isoxazoles et isoxazolines à activité anticonvulsivante, procédé de préparation et compositions thérapeutiques les contenant.**

(30) Priorité: **30.11.88 FR 8815718**

(43) Date de publication de la demande:
**06.06.90 Bulletin 90/23**

(45) Mention de la délivrance du brevet:
**24.11.93 Bulletin 93/47**

(84) Etats contractants désignés:
**AT BE CH DE ES FR GB GR IT LI LU NL SE**

(56) Documents cités:
**EP-A- 0 048 162**
**FR-A- 2 073 284**
**FR-A- 2 315 923**
**US-A- 2 126 329**

**BULLETIN DE LA SOCIETE CHIMIQUE DE FRANCE, 1963, pages 140-142, Paris, FR; R. PAUL et al.: "Oxydes de nitriles. II. Synthèse d'isoxazolines à partir du nitrométhane"**

**CHEMICAL ABSTRACTS, vol. 70, no. 11, 17 mars 1969, pages 323-324, résumé no. 47343z, Columbus, Ohio, US; G. BIANCHI et al.: "2-isoxazolines. II. Reactions of 5-acyl-2-isoxazolines"**

(73) Titulaire: **NOVAPHARME**
**23 rue Jonquoy**
**F-75014 Paris(FR)**

(72) Inventeur: **Lepage, Francis**
**13 rue du Général de Larminat**
**F-94000 Créteil(FR)**
Inventeur: **Hublot, Bernard**
**22 rue du Champ de Mars**
**F-75007 Paris(FR)**

(74) Mandataire: **Polus, Camille et al**
**c/o Cabinet Lavoix**
**2, Place d'Estienne d'Orves**
**F-75441 Paris Cedex 09 (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.6/3.3.1)

## Description

La présente invention se rapporte de manière générale à de nouveaux dérivés hétérocycliques doués d'activité anticonvulsivante, un procédé de préparation ainsi qu'à des compositions thérapeutiques les contenant.

On dispose d'un nombre relativement faible d'agents ayant une activité anticonvulsivante. Parmi ceux-ci un grand nombre présentent des inconvénients liés à des phénomènes d'échappement thérapeutiques, des effets secondaires gênants tels que diminution de la vigilance, somnolence, .. ou des effets toxiques, en particulier hépatotoxiques.

Voir à cet égard Adverse Reactions to Antiepileptic Drugs, Epilepsia 27(4), 323-330 (1986); S. Horowitz et coll., Hepatotoxic Reactions Associated with Carbamazepine Therapy, Epilepsia 29(2), 149-154 (1988); E.A. Swinyard et coll., The Effect of Chronic Felbamate Administration on Anticonvulsant Activity and Hepatic Drug-Metabolizing Enzymes in Mice and Rats, Epilesia, 28(3), 295-300 (1987).

Le but de la présente invention est de fournir de nouveaux composés ayant des propriétés anticonvulsivantes, et exempts des inconvénients de l'art antérieur.

Par ailleurs EP-A-0 048 162 décrit des produits insecticides consistant en des 3- et 5-isoxazolyl- ou 3-benzisoxazolyl-benzamides, comportant un substituant phényl (éventuellement halogéné) sur le groupe 3-isoxazolyle, et soit pas de substituant soit un ou deux substituant(s) phényle (substitué) sur le groupe 5-isoxazolyle.

La présente invention a ainsi pour objet des composés hétérocycliques de formule générale

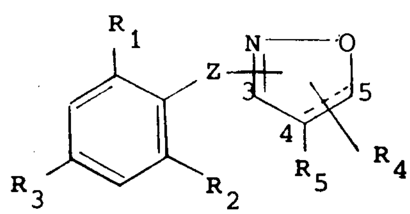

I

dans laquelle

$R_1$ et $R_2$ représentent chacun un groupe alkyle en $C_1$-$C_4$, phényle, benzyle, trifluorométhyle ou un atome d'halogène,

$R_3$ représente un atome d'hydrogène, un groupe hydroxy, un groupe alkyle ou alkoxy en $C_1$-$C_4$

$R_4$, en position 3 ou 5, représente un atome d'hydrogène, un groupe trifluorométhyle, ou un groupe alkyle, alkoxy ou hydroxyalkyle en $C_1$-$C_4$,

$R_5$ représente un atome d'hydrogène ou un groupe alkyle en $C_1$-$C_4$

ou $R_4$ et $R_5$ représentent ensemble un groupe tétramethylène

Z, en position 3 ou 5 sur l'hétérocycle, représente un groupe

-N($R_6$)-CO-,-CO-N($R_6$)-,-N($R_6$)-CO-N($R_6$)-, -CH($R_6$)-NH-CO-, ou -NH-CO-CH($R_6$)-,

dans lesquels $R_6$ représente un atome d'hydrogène ou un groupe alkyle en $C_1$-$C_4$;

la ligne pointillée représente la possibilité d'une seconde liaison, sous réserve que, lorsque Z est en position 5, $R_4$ et $R_5$ ne peuvent être simultanément un atome d'hydrogène.

Les composés (I) préférés sont particulièrement les isoxazoles.

Parmi les composés de formule générale I, on préfère encore ceux dans lesquels Z représente un groupe - N($R_6$)-CO- dans lequel $R_6$ représente un atome d'hydrogène.

Plus particulièrement, parmi les composés préférés selon l'invention on peut citer :

- le (diméthyl-2,6 phénylcarbamoyl)-3 méthyl-5 isoxazole
- le (diméthyl-2,6 phénylcarbamoyl)-5 méthyl-3 isoxazole
- le (diméthyl-2,6 phénylcarbamoyl)-3 hydroxyméthyl-5 isoxazole
- le (diméthyl-2,6 phénylcarbamoyl)-3 isoxazole
- le (triméthyl-2,4,6 phénylcarbamoyl)-3 méthyl-5 isoxazole
- le (méthyl-2 isopropyl-6 phénylcarbamoyl)-3 méthyl-5 isoxazole
- le N-(méthyl-5 isoxazolyl-3) diméthyl-2,6 benzamide
- le (chloro-2 méthyl-6 phénylcarbamoyl)-3 méthyl-5 isoxazole
- le (diméthyl-2,6 phénylcarbamoyl)-3 méthyl-5 isoxazoline.

Ces composés portent respectivement les numéros 1,16,29,6,11,14,19,13,4 dans les tableaux.

Selon un autre de ses aspects, la présente invention a pour objet un procédé de préparation des composés de formule générale I caractérisé en ce que l'on fait réagir un composé de formule générale

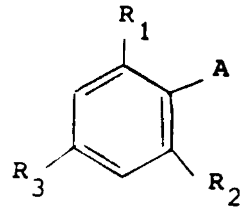

II

dans laquelle $R_1$, $R_2$ et $R_3$ ont les mêmes significations que dans la formule I et A représente un groupe -COOH,-COCl,-N($R_6$)H ou -CH($R_6$)-$NH_2$ dans lesquels $R_6$ représente un atome d'hydrogène ou un groupe alkyle en $C_1$-$C_4$, avec un composé de formule générale

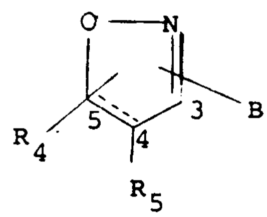

III

dans laquelle les symboles
$R_4$ et $R_5$ ont la même signification que dans la formule I et
B en position 3 ou 5 sur l'hétérocycle représente un groupement -COOH,-CH($R_6$)COOH,-COCl,-CH($R_6$)-COCl, ou -$NR_6$H dans lesquels $R_6$ représente un atome d'hydrogène ou un groupe alkyle en $C_1$-$C_4$ et
la ligne pointillée représente la possibilité d'une seconde liaison.
En particulier,

a) Quand Z représente les groupes -N($R_6$)-CO-, -CH($R_6$)-NH-CO- ou -NH-CO-CH($R_6$)-, $R_6$ étant tel que défini dans la formule générale I, ou condense une amine de formule générale II dans laquelle A représente le groupe -$NR_6$H ou -CH($R_6$)-$NH_2$ avec un acide ou un chlorure d'acide de formule générale III dans laquelle B représente un groupe -COOH,-CH-($R_6$)-COOH,-COCl ou -CH($R_6$)COCl.

Quand on utilise un acide de formule générale III, on effectue la réaction en présence de dicyclohexylcarbodiimide (DCC) ou carbonyldiimidazole (CDI) dans un solvant qui peut être le diméthylformamide (DMF) ou le tétrahydrofuranne (THF) à une température de 10 à25 ° C.

Dans le cas où l'on utilise un chlorure d'acide (III), on réalise la condensation en présence d'un capteur de proton tel que la triéthylamine ou le carbonate de potassium à une température de 20 à 120 ° C dans un solvant neutre tel que le toluène, l'acétone, etc.

b) Quand Z représente le groupe -$NR_6$-CO-$NR_6$, on condense un composé aniline de formule II dans laquelle A représente le groupe $NHR_6$ avec le phosgène pour former l'isocyanate correspondant qui est à son tour condensé avec une amine de formule III dans laquelle B représente $NR_6$H, dans un solvent neutre tel que le toluène ou le THF à une température de 20 à 120 ° C.

c) Quand Z représente le groupe -CO-N($R_6$)-, on condense un composé de formule générale II dans laquelle A représente -COOH ou -COCl avec un composé de formule générale III dans laquelle B représente -$NR_6$H. Cette réaction est effectuée de la même manière que celle sous a) ci-dessus.

Les acides de formule générale III sont connus de la littérature.

Il s'agit en particulier des acides :

- isoxazole carboxylique-3
- isoxazole carboxylique-3 substitué en position -5 par un méthyle, éthyle, isopropyle, tertbutyle,
- diméthyl-4,5 isoxazole carboxylique-3
- méthyl-3 isoxazole carboxylique-5
- tétrahydro-4,5,6,7 benzisoxazoline carboxylique-3
- méthyl-5 isoxazoline carboxylique-3
- diméthyl-4,5 isoxazoline carboxylique-3

- méthyl-5 isoxazolyl-3 acétique
- méthoxy-5 isoxazole carboxylique-3

L'acide trifluorométhyl-5 isoxazole carboxylique-3 a été préparé selon le procédé classique par condensation du trifluoroacétone et du diéthyle oxalate et ensuite condensation de la dicétone formée avec l'hydroxylamine.

Les amines de formule générale II sont toutes des produits disponibles dans le commerce ou décrits dans la littérature sauf pour celui ou A représente un groupe $CH(R_6)NH_2$ pour lequel la synthèse a été réalisée par réduction d'ester correspondant au moyen de $LiAlH_4$ suivi de la substitution de l'hydroxy par une fonction amine via le phthalimide correspondant.

d) Quand $R_4$ représente un groupe hydroxyalkyle en $C_1$-$C_4$, on peut aussi condenser un composé de formule générale

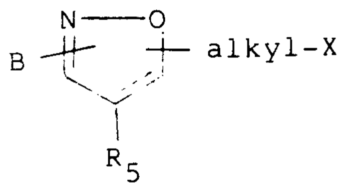

IV

dans laquelle X représente un halogène et B et $R_5$ possèdent la même signification que dans la formule III, avec un composé de formule II.

Ces composés sont ensuite traités par un carboxylate alcalin ou d'argent pour former les composés acyloxyalkyle ou benzoyloxyalkyle correspondants (formule I; $R_4$ = alkyle substitué par acyloxy ou benzoyloxy). Ces derniers sont ensuite hydrolysés en présence d'une base ou d'un acide pour former les composés hydroxyalkyle correspondants (formule I avec $R_4$ = hydroxyalkyle).

On peut également condenser un alcool propargylique ($HC=C-CH_2OH$) avec un composé de formule

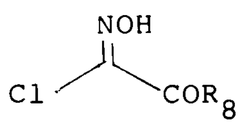

V

dans laquelle $R_8$ représente un groupe alkoxy pour former un composé de formule III dans laquelle $R_4$ représente un hydroxyalkyle et B et $R_5$ sont tels que définis. Ces composés sont ensuite hydrolysés et condensés selon le cas a) ci-dessus.

On peut aussi condenser l'alcool propargylique avec un composé de formule VI

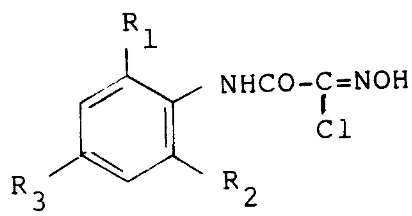

VI

dans laquelle $R_1$, $R_2$ et $R_3$ sont comme dans la formule I, pour former directement un composé de formule I dans laquelle $R_4$ représente un groupe hydroxyalkyle en $C_1$-$C_4$ et Z un groupe $-NR_6-CO$.

Certains composés de l'invention possèdent un ou plusieurs carbones assymétriques. Les isomères optiques correspondants font aussi partie de l'invention.

Les exemples suivants illustrent la préparation des composés de formule I.

EXEMPLE 1 : Préparation du (diméthyl-2,6 phénylcarbamoyl)-3 méthyl-5 isoxazole (composé 1)

On dissout 27g (0,2 mole) de diméthyl-2,6 aniline dans 100ml de toluène, on ajoute goutte à goutte 14,5g (0,1 mole) de chlorure d'acide méthyl-5 isoxazole carboxylique-3 sous agitation et on porte au reflux pendant 2h. On refroidit et on filtre le chlorhydrate formé. On reprend la phase organique par du bicarbonate de sodium à 10%. Après filtration et élimination du solvant, on recristallise dans du cyclohexane ou du pentane. F = 100°. Le rendement est d'environ 90%.

EXEMPLE 2 : Préparation du (diméthyl-2,6 phénylcarbamoyl)-3 éthyl-5 isoxazole (composé 2)

Sous azote, on dissout 5,1g (0.04 mole) d'acide éthyl-5 isoxazole carboxylique-3 dans 100ml de DMF, on ajoute 13g (0,08 mole) de carbonyl diimidazole et on laisse le mélange sous agitation pendant 2h à température ordinaire. On ajoute 9,7g (0,08 mole) de diméthyl-2,6 aniline et on laisse de nouveau sous agitation pendant 48h. Après filtration du précipité formé et évaporation du solvant, le résidu est cristallisé dans l'éther de pétrole. F = 82°. Le rendement est de 73%.

On obtient par l'une ou l'autre de ces méthodes (Ex. 1 ou 2) les composés 3 à 26,28,31.

EXEMPLE 3 : Préparation du N-(diméthyl-2,6 phényl)-N′(méthyl-5 isoxazolyl-3) urée (composé 27)

a) Préparation du diméthyl-2,6 phényl isocyanate.

On ajoute goutte à goutte 7g (0,05 mole) de diméthyl-2,6 aniline dissous dans 20 ml de toluène à une solution de phosgène à 20% dans le toluène (1,5 équivalents) maintenue à 0° par un bain de glace. Le mélange est ensuite porté au reflux pendant 3h. On concentre et distille le résidu sous vide. Eb = 100°/13 mmHg. Rendement : 76%.

b) Préparation du N-(diméthyl-2,6 phényl) N′-(méthyl-5 isoxazoyl-3) urée.

On porte au reflux pendant 16h 7g (0,04 mole) d'isocyanate précédent, 4g (0,04 mole)d'amino-3 méthyl-5 isoxazole dans un mélange toluène-THF (100-100ml). On filtre le précipité formé, concentre la solution. Les cristaux sont repris dans un mélange éthanol-eau. F = 200° - Rendement 64%.

EXEMPLE 4 : Préparation du composé N-(diméthyl-2,6 phénylcarbamoyl)-3 hydroxyméthyl-5 isoxazole (composé 29)

a) Préparation du (diméthyl-2,6 phénylcarbamoyl)-3 bromométhyl isoxazole.

On dissout 0,1 mole de chlorure de bromométhyl-5 isoxazole carbonyle-3 (J.C. Sircar et al, J. Org. Chem. 50, p. 5723-5727, 1985).

dans 200 ml de toluène. On ajoute 26g (0,2 mole) de diméthyl-2,6 aniline et porte au reflux pendant 5h. Après concentration du milieu réactionnel, le résidu est lavé par une solution de carbonate. Le résidu huileux est purifié par passage sur colonne (gel de silice). F° = 123-124°.

b) Préparation du (diméthyl-2,6 phénylcarbamoyl)-3 acétoxyméthyl-5 isoxazole.

On porte au reflux pendant 4h un mélange de 12,4g (0,04 mole) de composé bromométhyle précédemment préparé et 7,4g (0,044 mole) d'acétate d'argent dans 100 ml d'acide acétique. Après refroidissement on filtre le sel d'argent formé et concentre la solution. Le produit est purifié par passage sur colonne (gel de silice).

c) Préparation du (diméthyl-2,6 phénylcarbamoyl)-3 hydroxyméthyl-5 isoxazole.

On dissout le dérivé acétoxyméthyle précédant dans de l'éthanol, on ajoute 1,25 équivalent de solution de soude 10% et on porte le tout au reflux pendant 30 min. Après les traitements habituels le produit est purifié par passage sur colonne de silice (éluant $CH_2Cl_2$ puis $CH_2Cl_2$-$CH_3OH$ 95-5). Le rendement est de 40%- F° = 95°.

Le composé N° 30 peut être préparé de la même manière.

EXEMPLE 5 : Préparation du composé N-(diméthyl-2,6 benzyl carbamoyl)-3 méthyl-5 isoxazole (composé 28)

a) Préparation du diméthyl-2,6 benzyl alcool

On ajoute goutte à goutte en fonction du reflux, 17g d'ester diméthyl-2,6 benzoate d'éthyle (0,095 mole) dissous dans 50ml d'éther éthylique à une suspension de 3,72g (0,095 mole) de $LiAlH_4$ dans 100ml d'éther. L'introduction terminée, on porte au reflux durant 2h et laisse une nuit en contact. On décompose l'excès d'hydrure par introduction lente d'eau, (tout en refroidissant le ballon dans un bain d'eau glacée), puis de HCl 10%. Après les traitements habituels le produit est cristallisé de l'éther de pétrole. On obtient 10g de produit. F = 84°. Rendement 77,5%.

b) Préparation du diméthyl-2,6 benzyl phthalimide.

On dissout 10g d'alcool précédent (0,073 mole) dans 300 ml de THF et on ajoute 13,3g (0,09 mole) de phthalimido puis 22,9g (0,087 mole) de triphénylphosphine et ensuite goutte à goutte 13,9 ml de diéthylazodicarboxylate. La température s'élève jusqu'à 45°. On laisse ensuite en contact durant 24h

sous azote. On concentre le milieu réactionnel et purifie le résidu sur colonne de silice (éluant $CH_2Cl_2$). F = 154°. Rendement : 92,7%.

c) Préparation de la diméthyl-2,6 benzylamine.

On porte au reflux durant 8h 18g de dérivé phthalimido (0,068 mole), 13,5g (0,27 mole) d'hydrate d'hydrazine dans 200 ml d'éthanol. On filtre le solide obtenu, concentre la solution. Après traitements habituels le résidu est distillé. $E_{18mm}$ = 125-130°. Rendement : 66%.

d) Préparation du (diméthyl-2,6 benzyl-carbamoyl)-3 méthyl-5 isoxazole.

Selon l'exemple 1, en remplaçant la diméthyl-2,6 aniline par l'amine obtenue en c) ci-dessus.

Les composés de ces exemples, ainsi que d'autres composés de formule I sont rassemblés dans le tableau II, plus loin.

Les composés selon l'invention se sont révélés doués de propriétés intéressantes sur le système nerveux central, en particulier de propriétés anticonvulsivantes susceptibles de les rendre utiles dans le traitement de l'épilepsie ou comme complément de la thérapeutique anticomitiale, protecteur cérébral et augmentation de la mémoire.

Ainsi, l'invention comprend aussi les compositions thérapeutiques contenant à titre de principe actif les composés de formule générale I.

On donnera ci-après des résultats pharmacologiques et toxicologiques mettant en évidence les propriétés des composés de formule I.

## 1. Activité pharmacologique

L'activité anticonvulsivante est mesurée par les tests à l'électrochoc et au pentétrazole selon la technique de A. SWINYARD (ADD PROGRAM OF NINCDS BY H.J. KUPFERBERG E.A. SWINYARD AND G.D. GLADDING in advances in epileptology/XIIth Epilepsy International Symposium edited by M. DAM, L. GRAM and J.K. PENRY-RAVEN press NEW-YORK 1981). Les composés sont toujours administrés (au 1/10e de la DL50) par injections IP à des souris SWISS CD1 (Charles River) d'un poids moyen de 20-25g. Tous les produits sont dissous dans une solution 0,9% de chlorure de sodium ou mis en suspension dans une solution de carboxyméthyl-cellulose ou de tween à 1%.

Test à l'électrochoc. Des lots de 10 souris (1 lot témoin et un lot traité) sont utilisés pour chaque composé. Le lot traité reçoit le produit à tester par voie intrapéritonéale 30mn avant l'électrochoc. Celui-ci est appliqué à l'aide d'électrodes cornéennes (50 milliampères pendant 0,2 seconde). La protection est mesurée par le pourcentage d'animaux ne présentant pas d'extension des pattes postérieures.

Crises au pentétrazole. On injecte à des lots de 10 souris (1 lot témoin et un lot traité) 70 mg/kg s.c. de pentétrazole à raison de 0,2 ml/20g de poids corporel. Les produits à tester sont administrés 30 mn avant le pentétrazole par voie intrapéritonéale. Les animaux sont observés pendant 30 mn. Il est noté le nombre de crises cloniques supérieures ou égales à une durée de 5 secondes et le pourcentage des animaux protégés contre les crises cloniques.

Les résultats sont consignés dans le tableau ci-dessous.

## 2. Activité protection cérébrale

Cinq souris (20.25g) reçoivent une administration IP de produit ou de liquide véhicule 30 min avant d'être placées dans une enceinte close, où la pression atmosphérique est diminuée de 210 mm Hg. La durée de survie (en secondes) est mesurée depuis l'induction de l'hypoxie jusqu'à la disparition des mouvements respiratoires.

Le composé 1 (50 mg/kg) prolonge de manière significative le temps de survie : + 124%.

## 3. Détermination de la dose léthale 50

La toxicité est mesurée par la technique de MILLER et TAINTER par voie intrapéritonéale. Les résultats sont consignés dans le tableau ci-dessous.

| Résultats IP chez la souris | | | |
|---|---|---|---|
| Composés n° | DL50 mg/kg | % de protection pour | |
| | | Electrochoc | Pentétrazole |
| 1 | 375 | 100 | 30 |
| 2 | 375 | 60 | 20 |
| 3 | 500 | 100 | 40 |
| 5 | 500 | 70 | 80 |
| 6 | 500 | 80 | 50 |
| 7 | 750 | 90 | 40 |
| 11 | 750 | 100 | 50 |
| 12 | 500 | 100 | 60 |
| 13 | 1000 | 100 | 50 |
| 14 | 2000 | 100 | 30 |
| 16 | 750 | 100 | 90 |
| 19 | 2000 | 100 | 30 |
| 23 | | 40 | 60 |
| 29 | | 100 | |

Les compositions thérapeutiques selon l'invention peuvent être administrées par voie orale, parentérale ou endorectale.

Elles peuvent être sous la forme de comprimés, dragées, gélules, solutions ou suspensions injectables et suppositoires.

La quantité de principe actif administrée dépend évidemment du patient qui est traité, de la voie d'administration et de la sévérité de la maladie.

Cependant, la posologie quotidienne sera de l'ordre de 10 à 300 mg.

La dose unitaire peut être de 10 à 100 mg.

## EXEMPLES DE FORMULATION

1) Formule type comprimé :

Pour 5000 comprimés à 20 mg

| | |
|---|---|
| Composé de l'exemple 1 | 100g |
| Cellulose microcristalline | 1000g |
| Carboxyméthyl cellulose sodique | 15g |
| Stéarate de magnésium | 10g |
| | Total = 1125g |

- Mélanger tous les constituants dans un mélangeur Turbula$^{R}$ pendant 10min
- Comprimer sur machine alternative, poids théorique : 225 mg.

2) Formules type gélules :

Pour 5000 gélules taille 1 dosées à 10 mg

| | |
|---|---|
| Composé de l'exemple 1 | 50g |
| Amidon de maïs | 150g |
| Lactose | 1250g |
| PVP K30 | 75g |
| Talc | 30g |
| Stéarate de magnésium | 10g |
| | Total = 1565g |
| | Alcool à 50° = QS |

- Dans un mélangeur planétaire, mélanger pendant 10 min les constituants suivants : composé N° 1 - amidon de maïs - lactose - PVP.

- Toujours sous agitation, verser lentement l'alcool jusqu'à granulation satisfaisante.
- Etendre sur plateaux, faire sécher en étuve à 50 °C.
- Calibrer sur granulateur oscillant, grille de 1 mm.
- Mélanger le grain avec le talc et le stéarate de magnésium 10 min au Turbula[R].
- Mettre en gélule, poids théorique : 313 mg.

TABLEAU

| N° composé | Z (position) sur l'hétéro cycle | $R_1$ | $R_2$ | $R_3$ | $R_4$ | $R_5$ | Hétéro-cycle | F°C |
|---|---|---|---|---|---|---|---|---|
| 1 | -NH-CO- (3) | $CH_3$ | $CH_3$ | H | 5-$CH_3$ | H | isoxazole | 100° |
| 2 | -NH-CO- (3) | CH3 | $CH_3$ | H | 5-$CH_2CH_3$ | H | isoxazole | 82° |
| 3 | -NH-CO- (3) | $CH_3$ | $CH_3$ | H | 5-$CH_3$ | $CH_3$ | isoxazoline | 60° |
| 4 | -NH-CO- (3) | $CH_3$ | $CH_3$ | H | 5-$CH_3$ | H | isoxazoline | 92° |
| 5 | -NH-CO- (3) | $CH_3$ | $CH_3$ | H | 5-$OCH_3$ | H | isoxazole | 139° |
| 6 | -NH-CO- (3) | $CH_3$ | $CH_3$ | H | H | H | isoxazole | 61° |
| 7 | -NH-CO- (3) | $CH_3$ | $CH_3$ | H | 5-$CH(CH_3)_2$ | H | isoxazole | 96° |
| 8 | -NH-CO- (3) | $CH_3$ | $CH_3$ | H | 5-$C(CH_3)_3$ | H | isoxazole | 166° |
| 9 | -NH-CO- (3) | $CH_3$ | $CH_3$ | H | 5-$CF_3$ | H | isoxazole | |

<u>TABLEAU</u> (suite 1)

| N° composé | Z (position) sur l'hétéro cycle | $R_1$ | $R_2$ | $R_3$ | $R_4$ | $R_5$ | Hétéro-cycle | F°C |
|---|---|---|---|---|---|---|---|---|
| 10 | -NH-CO- (3) | $CH_3$ | $CH_3$ | H | 5- $CH_3$ | $CH_3$ | isoxazole | 123° |
| 11 | -NH-CO- (3) | $CH_3$ | $CH_3$ | $CH_3$ | 5-$CH_3$ | H | isoxazole | 106° |
| 12 | $CH_3$<br>\|<br>-N -CO- (3) | $CH_3$ | $CH_3$ | H | 5-$CH_3$ | H | isoxazole | 120° |
| 13 | -NH-CO- (3) | $CH_3$ | Cl | H | 5-$CH_3$ | H | isoxazole | 84° |
| 14 | -NH-CO- (3) | $CH_3$ | $CH_3$<br>\|<br>-CH<br>\|<br>$CH_3$ | H | 5-$CH_3$ | H | isoxazole | 112° |
| 15 | -NH-CO- (3) | $CH_3$<br>\|<br>-CH<br>\|<br>$CH_3$ | $CH_3$<br>\|<br>-CH<br>\|<br>$CH_3$ | H | 5-$CH_3$ | H | isoxazole | 161° |
| 16 | -NH-CO- (5) | $CH_3$ | $CH_3$ | H | 3-$CH_3$ | H | isoxazole | 120° |
| 17 | -NH-CO- (3) | $CH_3$ | $CH_3$ | H | $-(CH_2)_4-$ | | isoxazoline | 80° |

TABLEAU (suite II)

| N° composé | Z (position) sur l'hé-téro cycle | $R_1$ | $R_2$ | $R_3$ | $R_4$ | $R_5$ | Hétéro-cycle | F°C |
|---|---|---|---|---|---|---|---|---|
| 18 | $-NH-CO-CH_2-$<br>(3) | $CH_3$ | $CH_3$ | H | $5-CH_3$ | H | isoxazole | 124° |
| 19 | -CO-NH-<br>(3) | $CH_3$ | $CH_3$ | H | $5-CH_3$ | H | isoxazole | 180° |
| 20 | $CH_2CH_3$<br>-N-CO-<br>(3) | $CH_3$ | $CH_3$ | H | $5-CH_3$ | H | isoxazole | 72° |
| 21 | $CH(CH_3)_2$<br>-N-CO-<br>(3) | $CH_3$ | $CH_3$ | H | $5-CH_3$ | H | isoxazole | 82° |
| 22 | -CO-NH-<br>(5) | $CH_3$ | $CH_3$ | H | $3-CH_3$ | H | isoxazole | 133° |
| 23 | -NH-CO-<br>(5) | $CH_3$ | $CH_3$<br>-CH<br>$CH_3$ | H | $3-CH_3$ | H | isoxazole | 146° |
| 24 | $CH_3$<br>-N-CO-<br>(3) | $CH_3$ | $CH_3$<br>-CH<br>$CH_3$ | H | $5-CH_3$ | H | isoxazole | 72° |

TABLEAU (suite III)

| N° composé | Z (position) sur l'hétéro cycle | $R_1$ | $R_2$ | $R_3$ | $R_4$ | $R_5$ | Hétéro-cycle | F°C |
|---|---|---|---|---|---|---|---|---|
| 25 | -NH-CO- (3) | $CH_3$ | $CH_3$ | OH | 5-$CH_3$ | H | isoxazole | 143° |
| 26 | -NH-CO- (3) | $CH_3$ | $CH_3$ | $OCH_3$ | 5-$CH_3$ | H | isoxazole | 124° |
| 27 | -NH-CO-NH- (3) | $CH_3$ | $CH_3$ | H | 5-$CH_3$ | H | isoxazole | 200° |
| 28 | -$CH_2$NH-CO- (3) | $CH_3$ | $CH_3$ | H | 5-$CH_3$ | H | isoxazole | 99° |
| 29 | -NH-CO- (3) | $CH_3$ | $CH_3$ | H | 5-$CH_2$OH | H | isoxazole | 95° |
| 30 | -NH-CO- (3) | $CH_3$ | $CH_3$ | OH | 5-$CH_2$OH | H | isoxazole | 138° |
| 31 | -NH-CO (3) | $CH_3$ | $CH_3$ | Br | 5-$CH_3$ | H | isoxazole | 115° |

**Revendications**

**Revendications pour les Etats contractants suivants : AT, BE, CH, DE, FR, GB, IT, LI, LU, NL, SE**

**1.** Composés hétérocycliques de formule générale

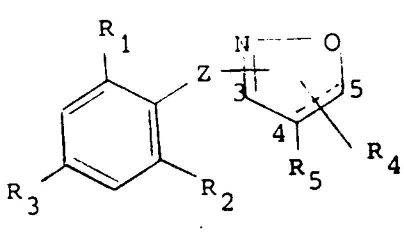

I

dans laquelle
$R_1$ et $R_2$ représentent chacun un groupe alkyle en $C_1$-$C_4$, phényle, benzyle, trifluorométhyle ou un atome d'halogène,
$R_3$ représente un atome d'hydrogène, un groupe hydroxy, un groupe alkyle ou alkoxy en $C_1$-$C_4$,
$R_4$, en position 3 ou 5, représente un atome d'hydrogène, un groupe trifluorométhyle, ou un groupe alkyle alkoxy ou hydroxyalkyle en $C_1$-$C_4$,
$R_5$ représente un atome d'hydrogène ou un groupe alkyle en $C_1$-$C_4$
ou $R_4$ et $R_5$ représentent ensemble un groupe tétraméthylène,
Z, en position 3 ou 5 sur l'hétérocycle, représente un groupe
-N($R_6$)-CO-,-CO-N($R_6$)-,-N($R_6$)-CO-N($R_6$)--CH($R_6$)-NH-CO-, ou -NH-CO-CH($R_6$)-,
dans lesquels $R_6$ représente un atome d'hydrogène ou un groupe alkyle en $C_1$-$C_4$;
la ligne pointillée représente la possibilité d'une seconde liaison, sous réserve que, lorsque Z est en position 5, $R_4$ et $R_5$ ne peuvent être simultanément un atome d'hydrogène.

2. Composés hétérocyliques selon la revendication 1, caractérisés en ce que l'hétérocycle est un isoxazole.

3. Composés hétérocycliques selon l'une des revendications 1 ou 2, caractérisé en ce que Z représente le groupe -N($R_6$)-CO- dans lequel $R_6$ représente un atome d'hydrogène.

4. Composé selon la revendication 1, caractérisé en ce qu'il consiste en le (diméthyl-2,6 phénylcarbamoyl)-3 méthyl-5 isoxazole.

5. Composé selon la revendication 1, caractérisé en ce qu'il consiste en le (diméthyl-2,6 phénylcarbamoyl)-5 méthyl-3 isoxazole.

6. Composé selon la revendication 1, caractérisé en ce qu'il consiste en le (diméthyl-2,6 phénylcarbamoyl)-3 hydroxyméthyl-5 isoxazole.

7. Composé selon la revendication 1, caractérisé en ce qu'il consiste en le (diméthyl-2,6 phénylcarbamoyl)-3 isoxazole.

8. Composé selon la revendication 1, caractérisé en ce qu'il consiste en le (triméthyl-2,4,6 phénylcarbamoyl)-3 méthyl-5 isoxazole.

9. Composé selon la revendication 1, caractérisé en ce qu'il consiste en le (méthyl-2-isopropyl-6 phénylcarbamoyl)-3 méthyl-5 isoxazole.

10. Composé selon la revendication 1, caractérisé en ce qu'il consiste en le N-(méthyl-5-isoxazolyl-3) diméthyl-2,6 benzamide.

11. Composé selon la revendication 1, caractérisé en ce qu'il consiste en le (chloro-2-méthyl-6 phénylcarbamoyl)-3 méthyl-5 isoxazole.

12. Composé selon la revendication 1, caractérisé en ce qu'il consiste en le (diméthyl-2,6 phénylcarbamoyl)-3 méthyl-5 isoxazoline.

13. Procédé de préparation d'un composé selon la revendication 1, caractérisé en ce que l'on fait réagir un composé de formule générale

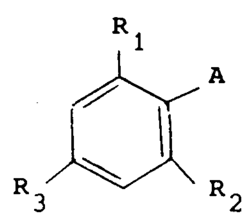

II

dans laquelle $R_1$, $R_2$ et $R_3$ ont les mêmes significations que dans la formumle I et A représente un groupe -COOH, -COCl,-N($R_6$)H ou -CH($R_6$)-$NH_2$ dans lesquels $R_6$ représente un atome d'hydrogène ou un groupe alkyle en $C_1$-$C_4$ avec un composé de formule générale

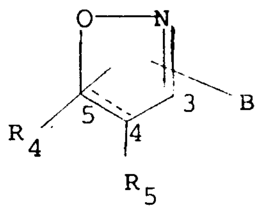

III

dans laquelle les symboles $R_4$ et $R_5$ ont la même signification que dans la formule I et
B en position 3 ou 5 sur l'hétérocycle représente un groupement -COOH,-CH($R_6$)COOH,-COCl,-CH($R_6$)-COCl, ou -$NR_6$H
dans lesquels $R_6$ représente un atome d'hydrogène ou un groupe alkyle en $C_1$-$C_4$ et
la ligne pointillée représente la possibilité d'une seconde liaison.

**14.** Procédé de préparation des composés de formule I dans laquelle Z représente les groupes -N($R_6$)-CO-,-CH($R_6$)-NH-CO- ou -NH-CO-CH($R_6$)- selon la revendication 13, caractérisé en ce que l'on condense une amine de formule générale II dans laquelle A représente le groupe -$NR_6$H, ou -CH($R_6$)-$NH_2$ avec un acide ou un chlorure d'acide de formule générale III dans laquelle B représente un groupe -COOH,-CH($R_6$)COOH,-COCl ou -CH($R_6$)COCl.

**15.** Composition thérapeutique, caractérisé en ce qu'elle contient à titre de principe actif un composé selon l'une quelconque des revendications 1 à 12.

**Revendications pour les Etats contractants suivants : ES, GR**

**1.** Procédé de préparation de composés hétérocycliques de formule générale

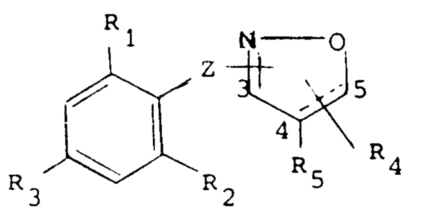

I

dans laquelle
$R_1$ et $R_2$ représentent chacun un groupe alkyle en $C_1$-$C_4$, phényle, benzyle, trifluorométhyle ou un atome d'halogène,
$R_3$ représente un atome d'hydrogène, un groupe hydroxy, un groupe alkyle ou alkoxy en $C_1$-$C_4$,
$R_4$, en position 3 ou 5, représente un atome d'hydrogène, un groupe trifluorométhyle, ou un groupe alkyle, alkoxy ou hydroxyalkyle en $C_1$-$C_4$,
$R_5$ représente un atome d'hydrogène ou un groupe alkyle en $C_1$-$C_4$
ou $R_4$ et $R_5$ représentent ensemble un groupe tétraméthylène,
Z, en position 3 ou 5 sur l'hétérocycle, représente un groupe
-N($R_6$)-CO-,-CO-N($R_6$)-,-N($R_6$)-CO-N($R_6$)--CH($R_6$)-NH-CO-, ou -NH-CO-CH($R_6$)-,
dans lesquels $R_6$ représente un atome d'hydrogène ou un groupe alkyle en $C_1$-$C_4$;
la ligne pointillée représente la possibilité d'une seconde liaison, sous réserve que, lorsque Z est en position 5, $R_4$ et $R_5$ ne peuvent être simultanément un atome d'hydrogène, caractérisé en ce que l'on fait réagir un composé de formule générale

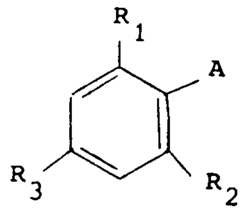

II

dans laquelle $R_1$, $R_2$ et $R_3$ ont les mêmes significations que dans la formule I et A représente un groupe -COOH,-COCl,-N($R_6$)H ou -CH($R_6$)-$NH_2$ dans lesquels $R_6$ représente un atome d'hydrogène ou un groupe alkyle en $C_1$-$C_4$ avec un composé de formule générale

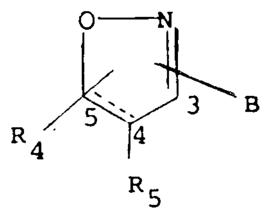

III

dans laquelle les symboles $R_4$ et $R_5$ ont la même signification que dans la formule I et
B en position 3 ou 5 sur l'hétérocycle représente un groupement -COOH,-CH($R_6$)COOH,-COCl,-CH($R_6$)-COCl, ou -$NR_6$H
dans lesquels $R_6$ représente un atome d'hydrogène ou un groupe alkyle en $C_1$-$C_4$ et
la ligne pointillée représente la possibilité d'une seconde liaison.

**2.** Procédé de préparation des composés de formule I dans laquelle Z représente les groupes -N($R_6$)-CO-,-CH($R_6$)-NH-CO- ou -NH-CO-CH($R_6$)- selon la revendication 1 , caractérisé en ce que l'on condense une amine de formule générale II dans laquelle A représente le groupe -$NR_6$H, ou -CH($R_6$)-$NH_2$ avec un acide ou un chlorure d'acide de formule générale III dans laquelle B représente un groupe -COOH,-CH($R_6$)COOH,-COCl ou -CH($R_6$)COCl.

**3.** Procédé selon la revendication 1 ou 2, caractérisé en ce que l'hétérocycle est un isoxazole.

**4.** Procédé selon l'une des revendications 2 ou 3, caractérisé en ce que Z représente le groupe -N($R_6$)-CO- dans lequel $R_6$ représente un atome d'hydrogène.

**5.** Procédé selon l'une des revendications 2 ou 3, caractérisé en ce que l'on prépare le (diméthyl-2,6 phénylcarbamoyl)-3 méthyl-5 isoxazole.

**6.** Procédé selon l'une des revendications 1 à 4, caractérisé en ce que l'on prépare le N-(méthyl-5-isoxazolyl-3) diméthyl-2,6 benzamide.

**7.** Procédé de préparation d'une composition thérapeutique, caractérisé en ce que l'on utilise à titre de principe actif un composé obtenu selon l'une quelconque des revendications 1 à 6.

## Claims

### Claims for the following Contracting States : AT, BE, CH, DE, FR, GB, IT, LI, LU, NL, SE

1. Heterocyclic compounds of general formula

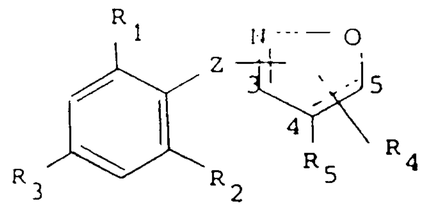

I

in which
$R_1$ and $R_2$ each represent a $C_1$-$C_4$ alkyl, phenyl, benzyl, trifluoromethyl group or a halogen atom,
$R_3$ represents a hydrogen atom, a hydroxy group, a $C_1$-$C_4$ alkyl or alkoxy group,
$R_4$, in position 3 or 5, represents a hydrogen atom, a trifluoromethyl group or a $C_1$-$C_4$ alkyl, alkoxy or hydroxyalkyl group,
$R_5$ represents a hydrogen atom or a $C_1$-$C_4$ alkyl group,
or $R_4$ and $R_5$ together represent a tetramethylene group,
Z, in position 3 or 5 on the heterocycle, represents a
-N($R_6$)-CO-, -CO-N($R_6$)-, N($R_6$)-CO-N($R_6$)--CH($R_6$)-NH-CO- or -NH-CO-CH($R_6$)- group,
in which $R_6$ represents a hydrogen atom or a $C_1$-$C_4$ alkyl group;
the dotted line represents the possibility of a second bond, subject to the fact that when Z is in position 5, $R_4$ and $R_5$ cannot simultaneously be a hydrogen atom.

2. Heterocyclic compounds according to claim 1, characterized in that the heterocycle is an isoxazole.

3. Heterocyclic compounds according to one of claims 1 or 2, characterized in that Z represents the -N-($R_6$)-CO- group in which $R_6$ represents a hydrogen atom.

4. Compound according to claim 1, characterized in that it consists of 3-(2,6-dimethyl phenylcarbamoyl) 5-methyl isoxazole.

5. Compound according to claim 1, characterized in that it consists of 5-(2,6-dimethyl phenylcarbamoyl) 3-methyl isoxazole.

6. Compound according to claim 1, characterized in that it consists of 3-(2,6-dimethyl phenylcarbamoyl) 5-hydroxymethyl isoxazole.

7. Compound according to claim 1, characterized in that it consists of 3-(2,6-dimethyl phenylcarbamoyl) isoxazole.

8. Compound according to claim 1, characterized in that it consists of 3-(2,4,6-trimethyl phenylcarbamoyl) 5-methyl isoxazole.

9. Compound according to claim 1, characterized in that it consists of 3-(2-methyl 6-isopropyl phenylcarbamoyl) 5-methyl isoxazole.

10. Compound according to claim 1, characterized in that it consists of N-(5-methyl 3-isoxazolyl) 2,6-dimethyl benzamide.

11. Compound according to claim 1, characterized in that it consists of 3(2-chloro 6-methyl phenylcarbamoyl) 5-methyl isoxazole.

12. Compound according to claim 1, characterized in that it consists of 3-(2,6-dimethyl phenylcarbamoyl) 5-methyl isoxazoline.

**13.** Preparation process for a compound according to claim 1, characterized in that a compound of general formula

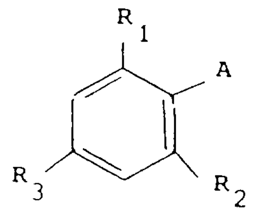

II

in which $R_1$, $R_2$ and $R_3$ have the same meanings as in formula I and A represents a -COOH, -COCl, -N-($R_6$)H or -CH($R_6$)-$NH_2$ group in which $R_6$ represents a hydrogen atom or a $C_1$-$C_4$ alkyl group, is reacted with a compound of general formula

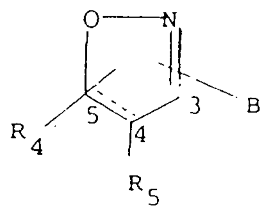

III

in which the $R_4$ and $R_5$ symbols have the same meaning as in general formula I and
B in position 3 or 5 on the heterocycle represents a -COOH, -CH($R_6$)COOH, -COCl, -CH($R_6$)COCl or -$NR_6$H group
in which $R_6$ represents a hydrogen atom or a $C_1$-$C_4$ alkyl group and the dotted line represents the possibility of a second bond.

**14.** Preparation process for the compounds of formula I in which Z represents the -N($R_6$(-CO-, -CH($R_6$)-NH-CO- or -NH-CO-CH($R_6$)- groups according to claim 13, characterized in that an amine of general formula II in which A represents the -$NR_6$H or -CH($R_6$)-$NH_2$ group is condensed with an acid or an acid chloride of general formula III in which B represents a -COOH, -CH($R_6$)COOH, -COCl or -CH($R_6$)COCl group.

**15.** Therapeutic composition, characterized in that it contains a compound according to any one of claims 1 to 12 as active ingredient.

**Claims for the following Contracting States : ES, GR**

**1.** Preparation process for heterocyclic compounds of general formula

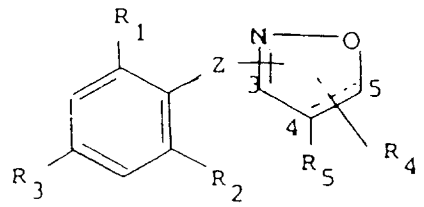

I

in which
$R_1$ and $R_2$ each represent a $C_1$-$C_4$ alkyl, phenyl, benzyl, trifluoromethyl group or a halogen atom,
$R_3$ represents a hydrogen atom, a hydroxy group, a $C_1$-$C_4$ alkyl or alkoxy group,
$R_4$, in position 3 or 5, represents a hydrogen atom, a trifluoromethyl group or a $C_1$-$C_4$ alkyl, alkoxy or

hydroxyalkyl group,
$R_5$ represents a hydrogen atom or a $C_1$-$C_4$ alkyl group,
or $R_4$ and $R_5$ together represent a tetramethylene group,
Z, in position 3 or 5 on the heterocycle, represents a
-N($R_6$)-CO-, -CO-N($R_6$)-, -N($R_6$)-CO-N($R_6$)--CH($R_6$)-NH-CO- or -NH-CO-CH($R_6$)- group,
in which $R_6$ represents a hydrogen atom or a $C_1$-$C_4$ alkyl group;
the dotted line represents the possibility of a second bond, subject to the fact that when Z is in position 5, $R_4$ and $R_5$ cannot simultaneously be a hydrogen atom, characterized in that a compound of general formula

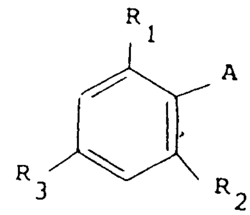

II

in which $R_1$, $R_2$ and $R_3$ have the same meanings as in formula I and A represents a -COOH, -COCl, -N($R_6$)H or -CH($R_6$)-$NH_2$ group in which $R_6$ represents a hydrogen atom or a $C_1$-$C_4$ alkyl group, is reacted with a compound of general formula

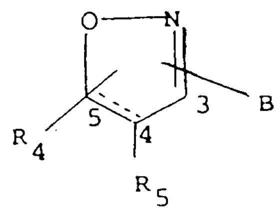

III

in which the $R_4$ and $R_5$ symbols have the same meaning as in general formula I and
B in position 3 or 5 on the heterocycle represents a -COOH, -CH($R_6$)COOH, -COCl, -CH($R_6$)COCl or -$NR_6$H group
in which $R_6$ represents a hydrogen atom or a $C_1$-$C_4$ alkyl group and the dotted line represents the possibility of a second bond.

**2.** Preparation process for the compounds of formula I in which Z represents the -N($R_6$(-CO-, -CH($R_6$)-NH-CO- or -NH-CO-CH($R_6$)- groups according to claim 1, characterized in that an amine of general formula II in which A represents the -$NR_6$H or -CH($R_6$)-$NH_2$ group is condensed with an acid or an acid chloride or general formula III in which B represents a -COOH, -CH($R_6$)COOH, -COCl or -CH($R_6$)COCl group.

**3.** Process according to claim 1 or 2, characterized in that the heterocycle is an isoxazole.

**4.** Process according to one of claims 2 or 3, characterized in that Z represents the -N($R_6$)-CO- group in which $R_6$ represents a hydrogen atom.

**5.** Process according to one of claims 2 or 3, characterized in that 3-(2,6-dimethyl phenylcarbamoyl) 5-methyl isoxazole is prepared.

**6.** Process according to one of claims 1 to 4, characterized in that N-(5-methyl 3-isoxazolyl) 2,6-dimethyl benzamide is prepared.

**7.** Preparation process for a therapeutic composition, characterized in that a compound obtained according to any one of claims 1 to 6 is used as active ingredient.

**Patentansprüche**

**Patentansprüche für folgende Vertragsstaaten : AT, BE, CH, DE, FR, GB, IT, LI, LU, NL, SE**

1. Heterocyclische Verbindungen der allgemeinen Formel

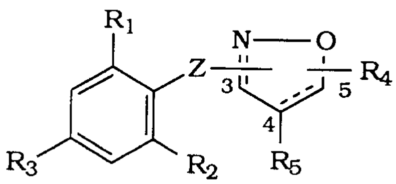

I

in der
$R_1$ und $R_2$ jeweils eine $C_1$-$C_4$-Alkylgruppe, eine Phenylgruppe, eine Benzylgruppe, eine Trifluormethylgruppe oder ein Halogenatom,
$R_3$ ein Wasserstoffatom, eine Hydroxylgruppe, eine $C_1$-$C_4$-Alkylgruppe oder eine $C_1$-$C_4$-Alkoxygruppe,
$R_4$ in der 3- oder in der 5-Stellung ein Wasserstoffatom, eine Trifluormethylgruppe, eine $C_1$-$C_4$-Alkylgruppe, eine $C_1$-$C_4$-Alkoxygruppe oder eine $C_1$-$C_4$-Hydroxyalkylgruppe,
$R_5$ ein Wasserstoffatom oder eine $C_1$-$C_4$-Alkylgruppe
oder $R_4$ und $R_5$ gemeinsam eine Tetramethylengruppe,
Z in der 3- oder 5-Stellung des Heterocyclus eine Gruppe der Formel
-N($R_6$)-CO-, -CO-N($R_6$)-, -N($R_6$)-CO-N($R_6$)--CH($R_6$)-NH-CO- oder -NH-CO-CH($R_6$)-
worin $R_6$ ein Wasserstoffatom oder eine $C_1$-$C_4$-Alkylgruppe darstellt, und die gestrichelte Linie die Möglichkeit einer zweiten Bildung mit der Maßgabe bedeuten, daß, wenn Z in der 5-Stellung steht, $R_4$ und $R_5$ nicht gleichzeitig Wasserstoffatome bedeuten.

2. Heterocyclische Verbindungen nach Anspruch 1, **dadurch gekennzeichnet**, daß der Hetercyclus ein Isoxazolring ist.

3. Heterocyclische Verbindungen nach einem der Ansprüche 1 oder 2, **dadurch gekennzeichnet**, daß Z eine Gruppe der Formel -N($R_6$)-CO- darstellt, worin $R_6$ ein Wasserstoffatom bedeutet.

4. Verbindung nach Anspruch 1, nämlich 3-(2,6-Dimethyl-phenylcarbamoyl)-5-methyl-isoxazol.

5. Verbindung nach Anspruch 1, nämlich 3-(2,6-Dimethyl-phenylcarbamoyl)-3-methyl-isoxazol.

6. Verbindung nach Anspruch 1, nämlich 3-(2,6-Dimethyl-phenylcarbamoyl)-5-hydroxymethyl-isoxazol.

7. Verbindung nach Anspruch 1, nämlich 3-(2,6-Dimethyl-phenylcarbamoyl)-isoxazol.

8. Verbindung nach Anspruch 1, nämlich 3-(2,4,6-Trimethyl-phenylcarbamoyl)-5-methyl-isoxazol.

9. Verbindung nach Anspruch 1, nämlich 3-(2Methyl-6-isopropyl-phenylcarbamoyl)-5-methyl-isoxazol.

10. Verbindung nach Anspruch 1, nämlich N-(5-Methyl-isoxazol-3-yl)-2,6-dimethyl-benzamid.

11. Verbindung nach Anspruch 1, nämlich 3-(2-Chlor-6-methyl-phenylcarbamoyl)-5-methyl-isoxazol.

12. Verbindung nach Anspruch 1, nämlich 3-(2,6-Dimethyl-phenylcarbamoyl)-5-methyl-isoxazolin.

13. Verfahren zur Herstellung einer Verbindung nach Anspruch 1, **dadurch gekennzeichnet,** daß man eine Verbindung der allgemeinen Formel

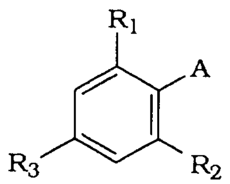

II

in der $R_1$, $R_2$ und $R_3$ die bezüglich der Formel I angegebenen Bedeutungen besitzen und A eine Gruppe der Formel -COOH, -COCl, -N($R_6$)H oder -CH($R_6$)-$NH_2$, worin $R_6$ ein Wasserstoffatom oder eine $C_1$-$C_4$-Alkylgruppe darstellt, bedeutet,
mit einer Verbindung der allgemeinen Formel

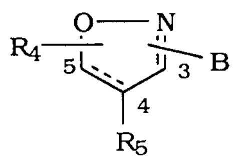

III

in der die Symbole $R_4$ und $R_5$ die bezüglich der Formel I angegebenen Bedeutungen besitzen, und
B in der 3- oder 5-Stellung des Heterocyclus eine Gruppe der Formel -COOH, -CH($R_6$)COOH, -COCl, -CH($R_6$)COCl oder -$NR_6$H,
worin $R_6$ ein Wasserstoffatom oder eine $C_1$-$C_4$-Alkylgruppe darstellt und die gestrichelte Linie die Möglichkeit einer weiteren Bindung bedeuten, umsetzt.

**14.** Verfahren nach Anspruch 13 zur Herstellung von Verbindungen der Formel I, worin Z eine Gruppe der Formel -N($R_6$)-CO-, -CH($R_6$)-NH-CO- oder -NH-CO-CH($R_6$)- darstellt, **dadurch gekennzeichnet,** daß man ein Amin der allgemeinen Formel II, in der A eine Gruppe der Formel -$NR_6$H oder -CH($R_6$)-$NH_2$ darstellt, mit einer Säure oder einem Säurechlorid der allgemeinen Formel III, in der B eine Gruppe der Formel -COOH, -CH($R_6$)COOH, -COCl oder -CH($R_6$)COCl bedeutet, kondensiert.

**15.** Therapeutische Zusammensetzung, **dadurch gekennzeichnet**, daß sie als Wirkstoff eine Verbindung nach einem der Ansprüche 1 bis 12 enthält.

**Patentansprüche für folgende Vertragsstaaten : ES, GR**

**1.** Verfahren zur Herstellung von heterocyclischen Verbindungen der allgemeinen Formel

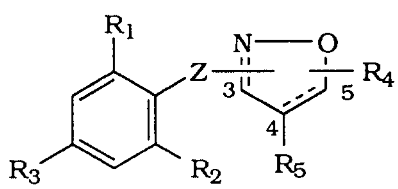

I

in der
$R_1$ und $R_2$ jeweils eine $C_1$-$C_4$-Alkylgruppe, eine Phenylgruppe, eine Benzylgruppe, eine Trifluormethylgruppe oder ein Halogenatom,
$R_3$ ein Wasserstoffatom, eine Hydroxylgruppe, eine $C_1$-$C_4$-Alkylgruppe oder eine $C_1$-$C_4$-Alkoxygruppe,
$R_4$ in der 3- oder in der 5-Stellung ein Wasserstoffatom, eine Trifluormethylgruppe, eine $C_1$-$C_4$-Alkylgruppe, eine $C_1$-$C_4$-Alkoxygruppe oder eine $C_1$-$C_4$-Hydroxyalkylgruppe,
$R_5$ ein Wasserstoffatom oder eine $C_1$-$C_4$-Alkylgruppe
oder $R_4$ und $R_5$ gemeinsam eine Tetramethylengruppe,
Z in der 3- oder 5-Stellung des Heterocyclus eine Gruppe der Formel
-N($R_6$)-CO-, -CO-N($R_6$)-, -N($R_6$)-CO-N($R_6$)--CH($R_6$)-NH-CO- oder -NH-CO-CH($R_6$)-

worin $R_6$ ein Wasserstoffatom oder eine $C_1$-$C_4$-Alkylgruppe darstellt, und die gestrichelte Linie die Möglichkeit einer zweiten Bildung mit der Maßgabe bedeuten, daß, wenn Z in der 5-Stellung steht, $R_4$ und $R_5$ nicht gleichzeitig Wasserstoffatome bedeuten, **dadurch gekennzeichnet**, daß man eine Verbindung der allgemeinen Formel

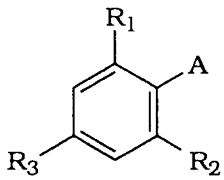

II

in der $R_1$, $R_2$ und $R_3$ die bezüglich der Formel I angegebenen Bedeutungen besitzen und A eine Gruppe der Formel -COOH, -COCl, -N($R_6$)H oder -CH($R_6$)-$NH_2$, worin $R_6$ ein Wasserstoffatom oder eine $C_1$-$C_4$-Alkylgruppe darstellt, bedeutet,
mit einer Verbindung der allgemeinen Formel

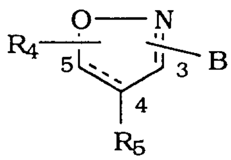

III

in der die Symbole $R_4$ und $R_5$ die bezüglich der Formel I angegebenen Bedeutungen besitzen, und
B in der 3- oder 5-Stellung des Heterocyclus eine Gruppe der Formel -COOH, -CH($R_6$)COOH, -COCl, -CH($R_6$)COCl oder -$NR_6$H,
worin $R_6$ ein Wasserstoffatom oder eine $C_1$-$C_4$-Alkylgruppe darstellt und die gestrichelte Linie die Möglichkeit einer weiteren Bindung bedeuten, umsetzt.

2. Verfahren nach Anspruch 1 zur Herstellung von Verbindungen der Formel I, worin Z eine Gruppe der Formel -N($R_6$)-CO-, -CH($R_6$)-NH-CO- oder -NH-CO-CH($R_6$)- darstellt, **dadurch gekennzeichnet**, daß man ein Amin der allgemeinen Formel II, in der A eine Gruppe der Formel -$NR_6$H oder -CH($R_6$)-$NH_2$ darstellt, mit einer Säure oder einem Säurechlorid der allgemeinen Formel III, in der B eine Gruppe der Formel -COOH, -CH($R_6$)COOH, -COCl oder -CH($R_6$)COCl bedeutet, kondensiert.

3. Verfahren nach Anspruch 1 oder 2, **dadurch gekennzeichnet**, daß der Heterocyclus ein Isoxazolring ist.

4. Verfahren nach einem der Ansprüche 2 oder 3, **dadurch gekennzeichnet**, daß Z die Gruppe der Formel -N($R_6$)-CO- darstellt, worin $R_6$ ein Wasserstoffatom bedeutet.

5. Verfahren nach einem der Ansprüche 2 oder 3, **dadurch gekennzeichnet,** daß man 3-(2,6-Dimethylphenylcarbamoyl)-5-methyl-isoxazol herstellt.

6. Verfahren nach einem der Ansprüche 1 bis 4, **dadurch gekennzeichnet**, daß man N-(5-Methylisoxazol-3-yl)-2,6-dimethyl-benzamid herstellt.

7. Verfahren zur Herstellung einer pharmazeutischen Zusammensetzung, **dadurch gekennzeichnet**, daß man als Wirkstoff eine Verbindung, erhalten nach einem der Ansprüche 1 bis 6, verwendet.